# EUROPEAN PATENT SPECIFICATION

(11) **EP 4 583 799 B1**
(45) Date of publication and mention of the grant of the patent: **25.03.2026**
(21) Application number: 23790506.2
(22) Date of filing: 21.09.2023
(51) Int. Cl.: A61B 18/14

(54) **SEALER, DIVIDER AND DISSECTOR DEVICE JAWS**
SIEGEL-, TRENN- UND DISSEKTORVORRICHTUNGSBACKEN
MÂCHOIRES DE DISPOSITIF DE SCELLEMENT, DE DIVISION ET DE DISSECTION

(30) Priority: 23.09.2022 US 202263376948 P
(43) Date of publication of application: 16.07.2025
(73) Proprietor: Bolder Surgical, LLC, Marlborough, MA 01752 (US)
(72) Inventor: COLE, Clayton, Marlborough, Massachusetts 01752 (US); CROWLEY, Tim, Marlborough, Massachusetts 01752 (US); SMITH, Ryan, Marlborough, Massachusetts 01752 (US); MAROM, Liad, Marlborough, Massachusetts 01752 (US)
(74) Representative: Gillespie, Richard
(86) International application number: PCT/US2023/074807
(87) International publication number: WO 2024/064840

(56) References cited:
- ES-T3- 2 240 723
- US-A1- 2011 184 404
- US-A1- 2013 079 774
- US-A1- 2013 310 832
- US-A1- 2014 100 568
- US-A1- 2021 022 798

## Description

### FIELD

This invention is related to surgical systems and devices. Specifically, but not intended to limit the invention as defined in the claims, embodiments of the invention are related to surgical devices for sealing, dividing and dissecting tissue during surgical procedures.

### BACKGROUND

US2021022798A1 discloses electrosurgical instruments and, more particularly, to electrosurgical forceps including thermal cutting elements to facilitate tissue treatment.

Surgeons often perform procedures in small or compact spaces of the body, such as the head/neck area or other parts of the body, as for example, thyroid resection, parathyroid resection, and/or other resections. Small or compact areas in the body are tightly packed with structures such as nerves, arteries, thyroid, esophagus, muscles, and/or other narrow spaces that inhibit surgeon's ability to maneuver around during surgical procedures (e.g., open, general, or laparoscopic).

The medical device industry has strived to provide reliable means to seal and divide tissue in tight and narrow body areas, including blood vessels. In some cases, vessel sealers have been offered that require the surgeon to repeatedly switch between a sealer and a cutting device. This method requires the surgeon first insert a sealer into the area, such as through a lumen or trocar, to seal tissue, then remove the device, and then re-enter with a cutting device. This method slows surgical procedures, so it is preferable to provide the sealing, dividing and dissector capabilities in a single device.

Exemplary of single devices, include among others: a) devices having a vessel sealer (e.g., jaws) in combination with a mechanical cutting mechanism (i.e., knife), b) devices having harmonic seal and cut operations, which also employ mechanical energy, and c) devices having a vessel sealer with electronic (e.g., radiofrequency energy) cutting capability.

Known medical systems are used in surgical procedures that include a sealing, dividing and dissector in a single device. Such devices include those illustrated and described in commonly-owned U.S. Pat. No. 10,765,471 issued on September 8, 2020 ("hereafter '471 patent"). Those skilled in the art will further recognize that the sealing system may include features disclosed in commonly-owned US Pat. No. 9,144,455 issued on September 29, 2015.

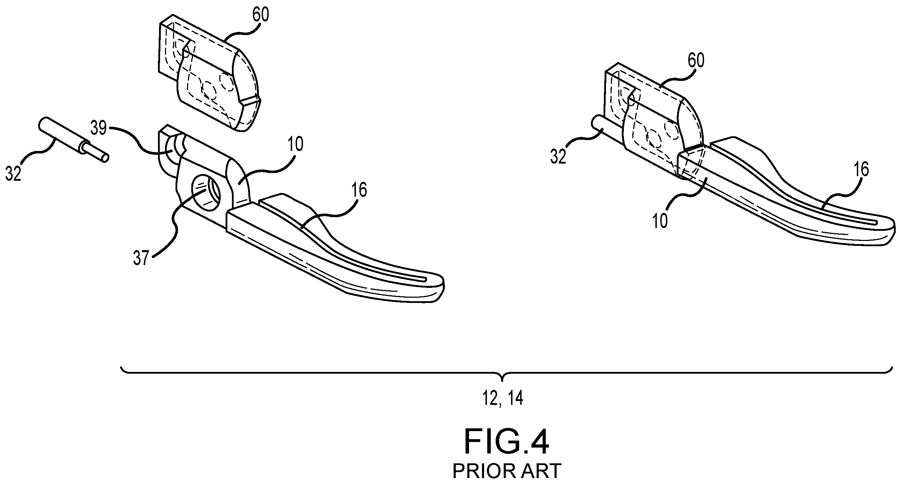

Additionally, known medical sealing, dividing and dissector devices include a variety of shapes of their elongated jaws (e.g., straight or curved configurations). Further, the prior art medical devices jaws 12, 14 are formed by known techniques, where the conductive material (e.g., metal or alloy) is manufactured in a single piece 10 (**FIG. 4**), and a non-conductive material (e.g., polymer or ceramic) covers a proximal portion of the jaws 12, 14 forming an over-mold 60. The unitary single piece 10 may include an aperture 37 configured to receive a pivot pin, and an aperture 39 configured to receive a cam-pin, a drive pin or boss. The unitary piece 10 is coupled to a wire 32 that provides an energy path through the jaws 12, 14.

In certain medical procedures, surgeons may use the sealing, dividing and dissector device for a prolong period of time, which can increase the temperature and material fatigue of the jaws (e.g., jaws 12, 14) and potentially lead to failure of the device. For example, the polymeric over-mold 60 and/or bushing (if any) can fatigue, melt and/or crack after extensive use of the device (e.g., prolonged heat exposure). In such cases, the compromised over-mold 60 or bushing can cause an electrical short, rendering the device not functional.

The teachings of the following U.S. Patents are considered relevant to this disclosure:
U.S. Pat. No. 5,876,401 to Schulze, U.S. Pat. No. 6,174,309 to Wrublewski, U.S. Pat. No. 6,458,128 to Schulze, U.S. Pat. No. 6,682,528 to Frazier, U.S. Pat. No. 7,083,618 to Couture, U.S. Pat. No. 7,101,373 to Dycus, U.S. Pat. No. 7,156,846 to Dycus, U.S. Pat. No. 7,101,371 to Dycus, U.S. Pat. No. 7,255,697 to Dycus, U.S. Pat. No. 7,722,607 to Dumbauld, U.S. Pat. No. 8,540,711 to Dycus, U.S. Pat. No. 7,131,971 to Dycus, U.S. Pat. No. 7,204,835 to Latterell, U.S. Pat. No. 7,211,080 to Treat, U.S. Pat. No. 7,473,253 to Dycus, U.S. Pat. No. 7,491,202 to Odom, U.S. Pat. No. 7,857,812 to Dycus, U.S. Pat. No. 8,241,284 to Dycus, U.S. Pat. No. 8,246,618 to Bucciaglia, U.S. Pat. No. 8,361,072 to Dumbauld, U.S. Pat. No. 8,469,956 to McKenna, U.S. Pat. No. 8,523,898 to Bucciaglia, U.S. Pat. No. 8,579,894 to Falkenstein, U.S. Pat. No. 8,968,311 to Allen, U.S. Pat. No. 9,011,437 to Woodruff, U.S. Pat. No. 9,028,495 to Mueller, U.S. Pat. No. 9,113,901 to Allen, U.S. Pat. No. 5,800,44 to Wales, U.S. Pat. No. 5,462,546 to Rydell, U.S. Pat. No. 5,445,638 to Rydell, U.S. Pat. No. 5,697,949 to Giurtino, U.S. Pat. No. 5,797,938 to Parashac, U.S. Pat. No. 6,334,860 to Dorn, U.S. Pat. No. 6,458,130 to Frazier, U.S. Pat. No. 6,113,598 to Baker, U.S. Pat. No. 6,033,399 to Gines, U.S. Pat. No. 9,737,357 to Dycus, U.S. Pat. No. 10,568,682 to Dycus, U.S. Pat. No. 9,375,263 to Allen, U.S. Pat. No. 9,456,863 to Moua, U.S. Pat. No. 7,604,635 to McClurken, U.S. Pat. No. 7,972,328 to Wham, and U.S. Pat. No. 7,655,007 to Baily.

The teachings of the following U.S. Patent Publications are also considered relevant to the present disclosure:
US2013/0131651 to Strobl, US2007/0173813 to Odom, and US2009/0076506 to Baker.

### SUMMARY OF THE INVENTION

The invention is defined in claim 1. Advantageous embodiments of the invention are defined in the dependent claims.

### SUMMARY OF THE DISCLOSURE

In one embodiment, an electrosurgical device, comprises a pair of opposing jaws including a first jaw and a second jaw, the first and second jaws configured to move between an open position and a tissue clamping position, each of the first and second jaws comprising an electrically conductive core member and an electrically conductive support member separated proximally from the core member by a gap; an electrically insulative material disposed in the gap such that the support member is substantially electrically isolated from the core member, and a pin, wherein the first and second jaw core members are pivotally coupled to the pin about which the first and second jaws rotate to move between the open position and the tissue clamping position.

In some embodiments, the electrosurgical device comprises an actuation mechanism operably coupled to the first and second jaw support members for enabling manipulation of the pair of opposing jaws.

In some embodiments, a surface section of at least one of the support members of the electrosurgical device has a length that at least partially defines the gap, and wherein the length is substantially orthogonal to a force vector exerted when a user manipulates the actuation mechanism. The force vector results in movement of the at least one support member relative to the respective core member. The relative movement of the at least one support member and the respective core member applies a compressive load on the electrically insulating material disposed in the gap. The gap is configured to spread the compressive load substantially uniformly along a length of the gap. The relative movement of the at least one support member and the respective core member applies a shear load on the electrically insulating material disposed in the gap, wherein the shear load is substantially less than compressive load. Optionally, the gap is non-linear.

In some embodiments, the pin is substantially electrically isolated from the respective first and second jaw core members. Optionally, one of a polymer over-mold, a polymer bushing and a ceramic bushing electrically isolates the pin from the respective conductive core members.

In some embodiments, the actuation mechanism comprises a cam-pin that moves through a cam slot. In other embodiments, the actuation mechanism comprises a first link coupled to the first jaw support member, a second link coupled to the second jaw support member and respective drive holes dispose within the first link and the second link.

In some embodiments, a polymer over-mold is partially disposed over the at least one support member and the respective core member.

In another embodiment, an electrosurgical device comprises a pair of opposing jaws including a first jaw and a second jaw, the first and second jaws configured to move between an open position and a tissue clamping position, each of the first and second jaws comprising an electrically conductive core member and an electrically conductive support member separated proximally from the core member by a gap; an electrically insulative material is disposed in the gap such that the support member is substantially electrically isolated from the core member; a pin, wherein the first and second jaws are pivotally coupled to the pin about which the first and second jaws rotate to move between the open position and the tissue clamping position; and a linkage mechanism disposed proximal to the pin and operably coupled to the respective first and second jaw support members for enabling manipulation of the pair of opposing jaws.

In yet another embodiment, an electrosurgical device comprises a pair of opposing jaws including a first jaw and a second jaw, the first and second jaws configured to move between an open position and a tissue clamping position, each of the first and second jaws comprising an electrically active core member with respect to electrical operation of the device and an electrically inactive support member disposed adjacent to the core member; a pin, wherein the first and second jaw core members are pivotally coupled to the pin about which the first and second jaws rotate to move between the open position and the tissue clamping position; and a linkage mechanism disposed proximal to the pin and operably coupled to the respective first and second jaw support members for enabling manipulation of the pair of opposing jaws.

In some embodiments, the linkage mechanism is electrically isolated from the pin and the first and second jaw core members. The linkage mechanism comprises a first link coupled to the first jaw support member, a second link coupled to the second jaw support member and respective drive holes dispose within the first link and the second link.

In some embodiments, a force vector is generated when a user manipulates the linkage mechanism that results in movement of the at least one support member relative to the respective core member.

In some embodiments, a surface section of at least one of the support members has a length that at least partially defines the gap, and wherein the length is substantially orthogonal to a force vector exerted when a user manipulates the linkage mechanism. The relative movement of the at least one support member and the respective core member applies a compressive load on the electrically insulating material disposed in the gap, and wherein the gap is configured to spread the compressive load substantially uniformly along a length of the gap. The relative movement of the at least one support member and the respective core member applies a shear load on the electrically insulating material disposed in the gap, wherein the shear load is substantially less than compressive load. Optionally, the gap is non-linear.

In some embodiments, the pin is substantially electrically isolated from the respective first and second jaw core members. One of a polymer over-mold, a polymer bushing and a ceramic bushing electrically isolates the pin from the respective conductive core members. A polymer over-mold is partially disposed over the at least one support member and the respective core member.

Other and further aspects and features of embodiments of the disclosed inventions will become apparent from the ensuing detailed description in view of the accompanying figures.

### BRIEF DESCRIPTION OF THE DRAWINGS

The drawings illustrate the design and utility of embodiments, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only exemplary embodiments and are not therefore to be considered limiting in the scope of the claims.
FIG. 1 is a perspective view of a medical system according to embodiments of the disclosed inventions;
FIGS. 2A-2B are side views of the end portion of the system of FIG. 1;
FIGS. 3A-3B are perspective views of an alternative end portion of the system of FIG. 1;
FIG. 4 is a perspective and exploded view of a prior art medical device jaw;
FIG. 5 is a perspective view of a medical device jaw, according to embodiments of the invention;
FIG. 6 is a perspective view of components of the medical device jaw of FIG. 5;
FIG. 7 is a perspective view of the components of the medical device jaw of FIG. 5;
FIG. 8 is a side view of the components of the medical device jaw of FIG. 5;
FIG. 9 is a side view of the medical device jaw of FIG. 5 with an over-mold depicted as transparent to better appreciate the jaw, and further including a graph of a force vector and a section of the jaw;
FIG. 10 is a partial sectional view of the medical device jaw of FIG. 5;
FIG. 11 is a cross-sectional partial view of an alternative embodiment of a medical device jaw; and
FIG. 12 is a cross-sectional partial view of another alternative embodiment of a medical device jaw.

### DETAILED DESCRIPTION

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. The recitation of numerical ranges by endpoints includes all numbers within that range (e.g., 1 to 5 includes 1, 1.5, 2, 2.75, 3, 3.80, 4, and 5). As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Various embodiments of the disclosed inventions are described hereinafter with reference to the figures. The figures are not necessarily drawn to scale, the relative scale of select elements may have been exaggerated for clarity, and elements of similar structures or functions are represented by like reference numerals throughout the figures. It should also be understood that the figures are only intended to facilitate the description of the embodiments, and are not intended as an exhaustive description of the invention or as a limitation on the scope of the invention, which is defined only by the appended claims. In addition, an illustrated embodiment needs not have all the aspects or advantages shown. An aspect or an advantage described in conjunction with a particular embodiment is not necessarily limited to that embodiment and can be practiced in any other embodiments even if not so illustrated

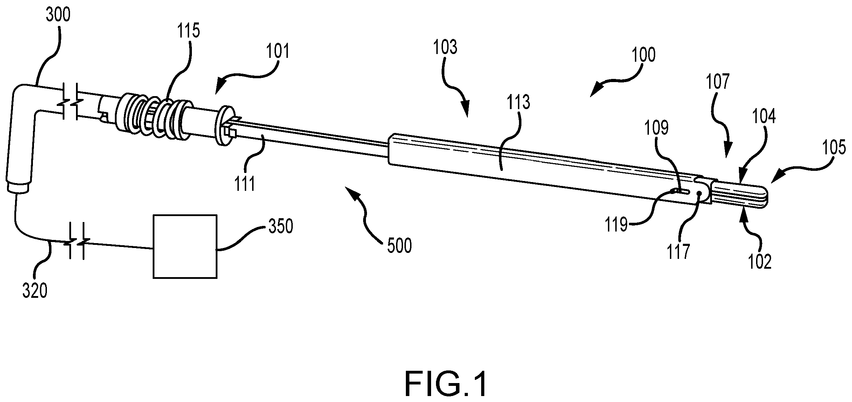

**FIG. 1** is a perspective view of a medical system 500, in accordance with embodiments of the disclosed inventions. The system 500 includes a device 100 having a proximal portion 101, a body portion 103 and a distal portion 107. The proximal portion 101 of the device is configured to be coupled to an actuator or actuation mechanism 300, such as a handle, a laparoscopic system, a surgical robotic system or the like. The body portion 103 of the medical device 100 comprises an inner member 111 (e.g., pull tube or pull rod) and an outer tubular member 113, where the inner member 111 is configured to translate relative to the outer tubular member 113. The distal portion 107 of the medical device 100 comprises a bipolar end effector 105 having a first jaw 102 and a second jaw 104. The actuation mechanism 300 is configured to actuate the medical device 100, control physical movements (e.g., open and close/clamping positions of the jaws 102, 104), engage the end effector 105 with tissue of a patient, and electrically engage the end effector 105 to seal and divide tissue. An electrical wire or wires 320 extend from a power source 350, such as a radio frequency generator, through the actuation mechanism 300 to the end effector 105; the electrical wires 320 are configured to deliver energy to the end effector 105. In an alternative embodiment, the electrical wires 320 extend from the actuation mechanism 300 to the end effector 105, and the connection of the medical device 100 to the power source 350 is wireless. The energy delivered may be radiofrequency (RF) energy or any other suitable energy for sealing and cutting tissue. The power source 350 (i.e., generator) is described in commonly-owned US Pat. No. 10,342,599 issued on July 9, 2019, systems and methods for providing power to electrosurgical devices is described in commonly-owned U.S. Pat. No. 9,039,694 issued on May 26, 2015 ("hereafter '694 patent"). Commonly-owned U.S. Pat. No. 9,265,561 issued on February 23, 2016 ("hereafter '561 patent") discloses a low power device having certain parameters for reliably and safely sealing tissue.

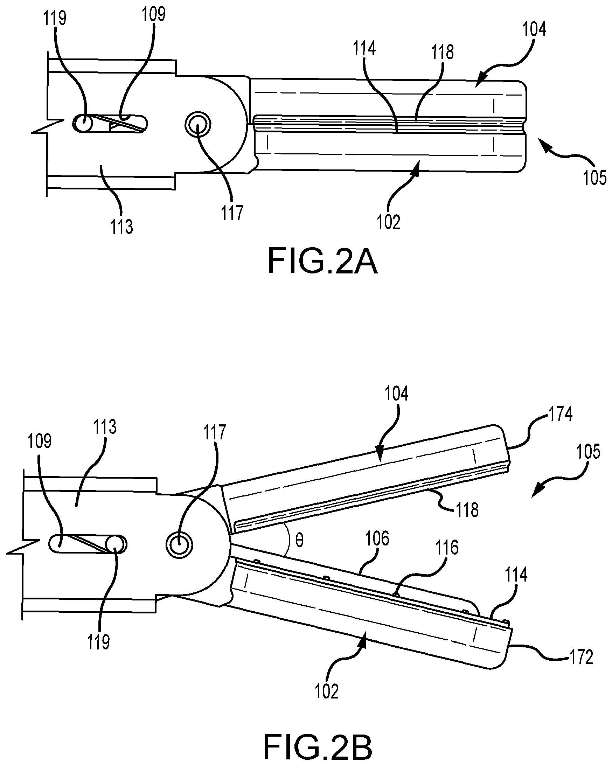

Referring back to the proximal portion 101 of the medical device 100, the proximal portion 101 includes a spring cartridge 115 (or other suitable resilient mechanism) configured to exert a spring-limited sealing force when actuated by the actuation mechanism 300, thereby compressing the spring and proximally translating (e.g., pulling) the inner member 111. The actuation mechanism 300 comprises a bolt or cam-pin 119 that moves through a cam slot 109 or multiple slots 109. A distal portion of the inner member 111 of the medical device 100 is fixedly attached the cam-pin 119, the ends of which pass through opposing slots 109 of the outer tubular member 113 (**FIGS. 1-2B**). The cam-pin 119 is slidably disposed within slots 109 and is configured to transfer forces to the end effector 105, as the inner member 111 longitudinally translates relative to outer tubular member 113, thereby allowing jaws 102 and 104 to transition between a closed, clamping or approximated position (**FIG. 2A**) and an opened position (**FIG. 2B**). The slot 109 of the outer tubular member 113 is configured to allow movement of the cam-pin 119 of the inner member 111 without transferring forces to the outer tubular member 113. The slot 109 of the outer tubular member 113 of the medical device 100 minimizes or avoids undesirable motion of the jaws 102 and 104 (e.g., rotation). The distal portion 107 of the medical device 100 further comprises a pivot pin 117 disposed between the jaws 102 and 104. The pivot pin 117 is configured to serve as a bearing surface for the jaws 102 and 104 and maintain alignment of the components of the end effector 105. The first jaw 102 and second jaw 104 are pivotally coupled to the pin 117 about which the first and second jaws 102, 104 rotate to move between the open position and the tissue clamping position.

**FIGS. 2A-B** are side views of the end effector 105 of the medical device 100 of the system 500, having jaws 102 and 104 in a closed (i.e., tissue clamping position) and opened configuration, respectively. As shown in **FIG. 2A****,** the cam-pin 119 is slidably disposed within a proximal end of the slot 109 of the outer tubular member 113 when the jaws 102 and 104 are in a clamping configuration. **FIG. 2B** shows the cam-pin 119 at a distal end of the slot 109 of the outer tubular member 113 when the jaws 102 and 104 are in an open configuration. Both jaws 102 and 104 move relative to the outer tubular member 113 to form the opened configuration of the end effector 105 of the medical device 100. In the opened configuration, the jaws 102 and 104 are configured to form an angle in a range of 20 to 100 degrees. **FIG. 2B** illustrates an exemplary opened configuration having an angle θ of approximately 25° between jaws 102 and 104. In some embodiments, the angle θ is approximately 60° between jaws 102 and 104 (not shown). In some embodiments, the length of the respective jaws 102 and 104 are in a range of 15 to 55 millimeters measured from the pivot pin 117 to the distal ends of each jaw (e.g., non-traumatic distal end portions 172 and 174 better shown in **FIG. 2B**). In an alternative embodiment, only one of the jaws moves relative to the outer tubular member 113 (not shown). Additionally, each jaw 102, 104 comprises a respective conductive seal surface 114, 118, as show in **FIGS. 2A-B****.** The seal surface 114 of the jaw 102 includes one or more non-conductive stop members 116 comprised of a ceramic or any other suitable non-conductive material, to prevent direct contact between the seal surfaces 114 and 118 to prevent an electrical short, in a manner known to those skilled in the art. In some embodiments, the seal surface 118 may also include one or more non-conductive stop members 116. The end effector 105 further comprises a cutting member 106 (e.g., knife, blade, electrode, electric knife, or their like), as shown in **FIG. 2B****.**

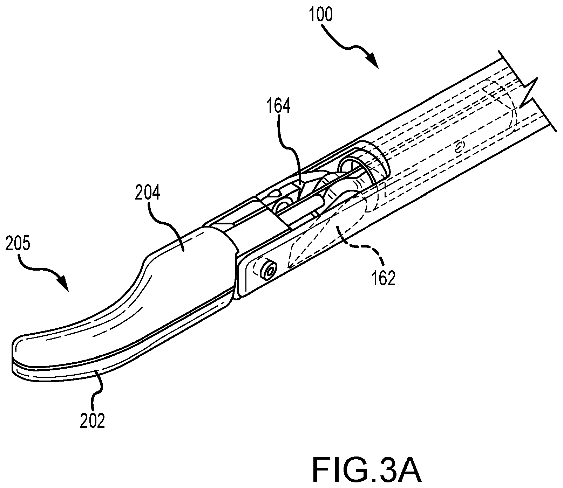

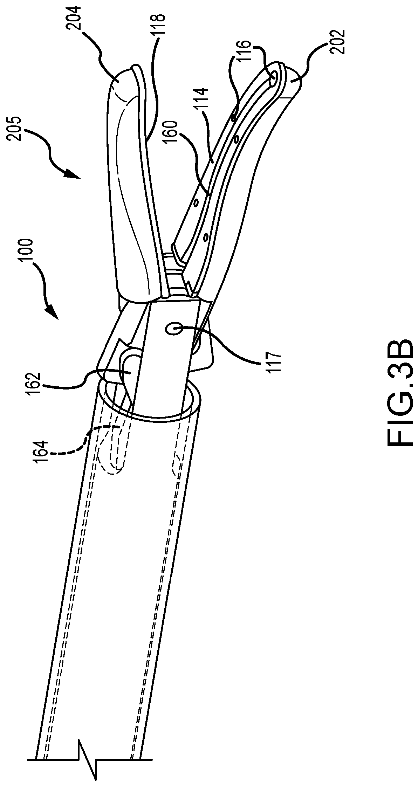

**FIGS. 3A-B** are perspective views of an alternative embodiment of an end effector 205 of the medical device 100, according to embodiments of the invention. The alternative end effector 205 of the medical device 100 of **FIGS. 3A-B** comprises a pair of opposing elongated jaws, including a first jaw 202 and a second jaw 204. The pair of opposing jaws 202 and 204 of **FIGS. 3A-B** are similar to the opposing jaws 102 and 104 of **FIGS. 1-2B****,** except that the jaws 202 and 204 are curved and thereby offset from the longitudinal axis A-A of the device 100, as shown in **FIG. 3A****.** The pair of opposing jaws 202, 204 also comprise respective conductive seal surfaces 114, 118, as show in **FIG. 3B****.** The seal surface 114 of the jaw 202 include one or more non-conductive stop members 116. The pair of jaws of either end effector 105, 205 may be composed of a suitable conductive material, metal, alloy or their like. Each of the jaws 102, 104, 202, 204 may comprise a straight or curved elongated channel, such as elongated channel 160 (**FIG. 3B**), that defines a path for the cutting element 106 to travel or be disposed within the end effector 105, 205 and cut tissue clamped or grasped by the respective pair of jaws 102, 104, or pair of jaws 202, 204 (not shown). Further, instead of the cam-pin 119 of **FIGS. 1-2B****,** the alternative end effector 205 of the medical device 100 comprises a first link 162 coupled to the first jaw 202 (e.g., first jaw support member) and a second link 164 coupled to the second jaw 204 (e.g., second jaw support member), such that the links 162 and 164 actuate the pair of jaws, in a manner similar to that described or illustrated in the '471 patent. The first link 162 and second link 164 comprise respective drive holes (not shown).

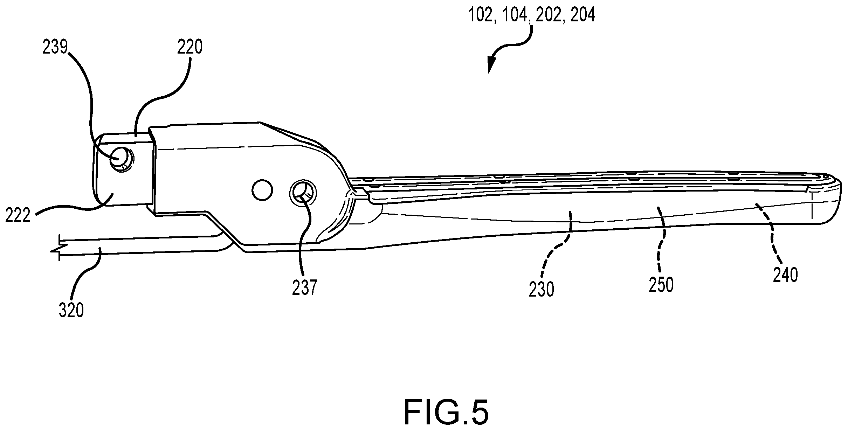

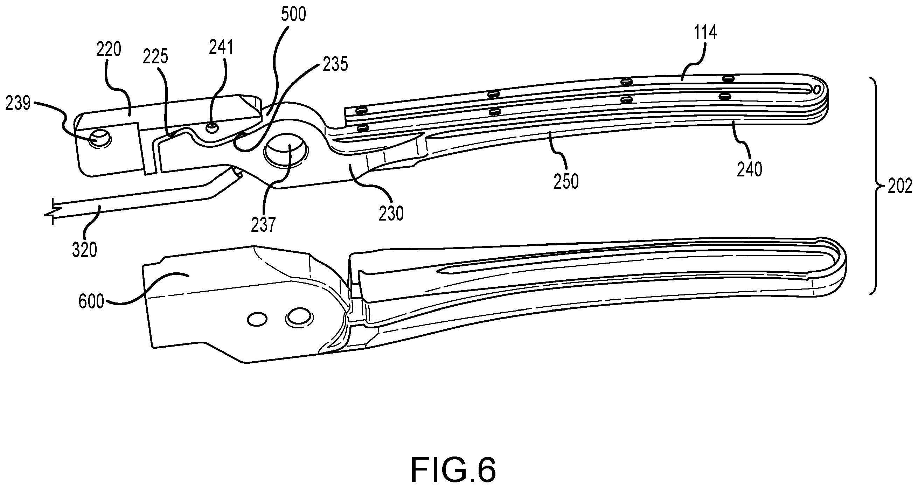

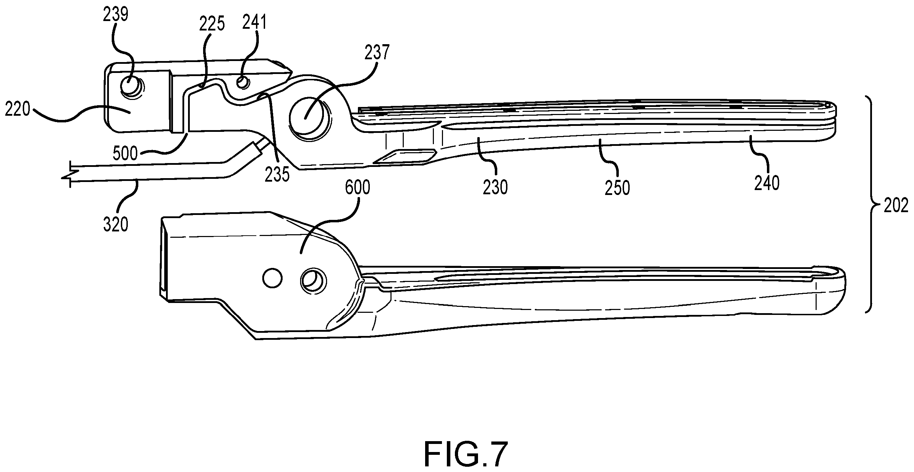

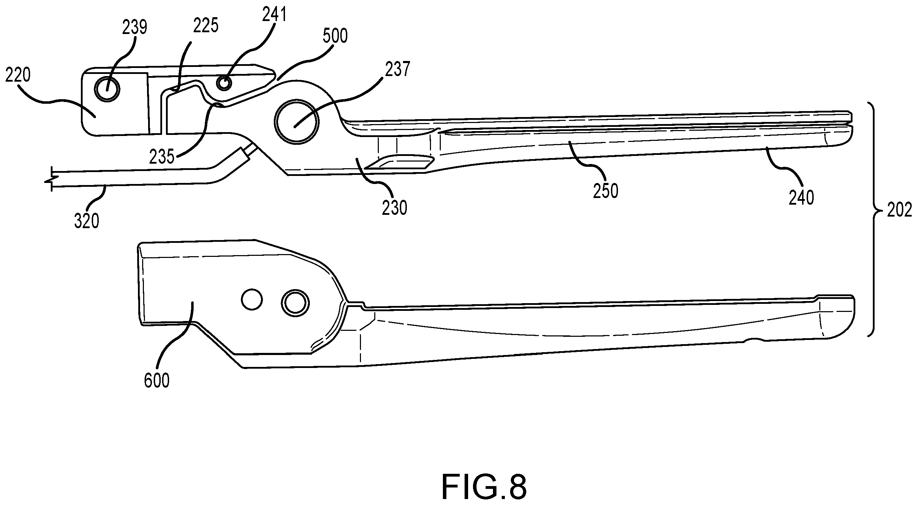

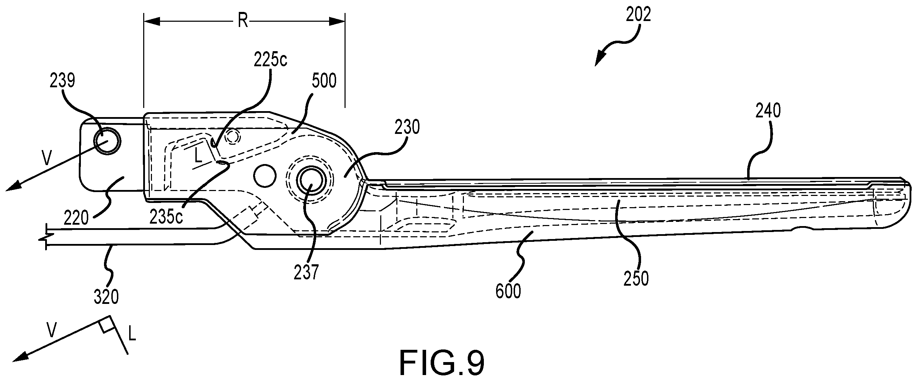

**FIG. 5** is a perspective view of either pair of opposing jaws 102/104, or pair of opposing jaws 202/204 of the effectors 105 or 205 of the medical device 100, according to embodiments of the invention. **FIGS. 6-7** are perspective views and **FIG. 8** is a side view of components of the either jaw of **FIG. 5****,** and **FIG. 9** is another side view of the jaw of **FIG. 5** with an over-mold depicted as transparent. For ease in illustration, **FIGS. 5-9** depicts the first jaw 202 but it should be appreciated that the description and depiction of the jaw 202 and its components, also applies to the opposing second jaw 204, or each of the jaws 102, 104, and its respective components. As shown in **FIGS. 6-9****,** the jaw 202 comprises a support member 220 (e.g., proximal portion or flag) and a core member 250; the core member 250 comprises an elongated body 230 and a distal portion 240. The core member 250 is composed of suitable electrically active conductive material (e.g., metals, alloys or combination thereof). The support member 220 is composed of conductive material (e.g., metals, alloys or combination thereof) that may be electrically active or electrically inactive. The support member 220, and the core member 250 of the jaw 202 are manufactured by techniques known by those skilled in the art, such as molding, extrusion or the like. As shown in **FIG. 6****,** the jaw 202 comprises a conductive surface 114 configured to seal tissue grasped, held or restrained between the jaws (not shown). The jaw 202 is coupled to the wire 320 (**FIGS. 6-9**) that provides an energy path from the energy source (**FIG. 1**) to the seal surface 114, through the core member 250 (e.g., elongated body 230 and/or distal portion 240). The wire 320 may be soldered or welded to the jaw 202 via an insulation-displacement contact or insulation piercing contact in a manner known to those skilled in the art.

As shown in **FIGS. 6-9****,** the support member 220 of the jaw 202 comprises a first aperture 239 configured to receive the cam-pin 119, previously described in **FIGS. 1-2B****.** The elongated body 230 portion of the jaw 202 comprises a second aperture 237 configured to receive the pivot pin 117, previously described in **FIGS. 1-2B****.** The elongated body 230 portion of core member 250 of the jaw 202 may comprise a third aperture 241, in a manner similar to that described or illustrated in the '471 patent. The first, second and third apertures 237, 239 and/or 241 may include non-conductive bushings, in a manner similar to that described or illustrated in the '471 patent. The apertures 237, 239 and/or 241 may comprise holes, slots, cavities or their like.

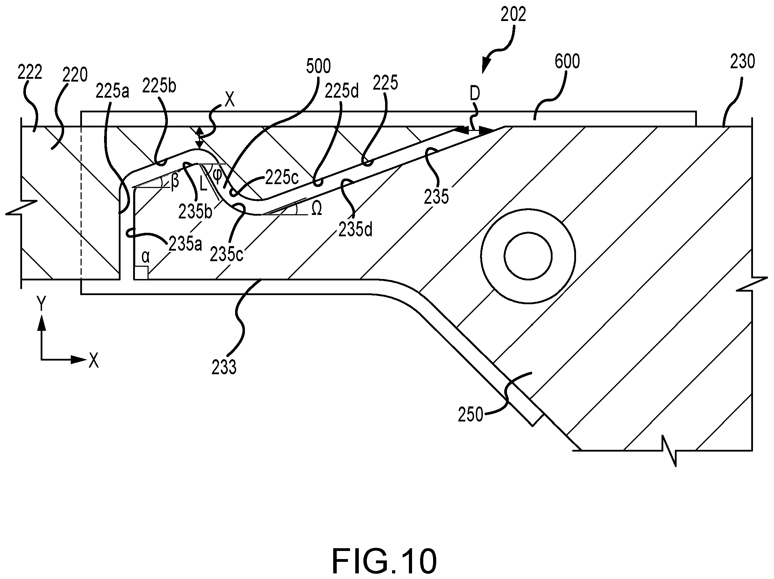

The jaw 202 further comprises a non-conductive over-mold 600, as shown in **FIGS. 6-10****.** In order to better appreciate the components of the jaw 202, the jaw 202 is depicted in an exploded view in **FIGS. 6-8****,** where the over-mold 600 is shown separate and detached from the support member 220, and core member 250 of the jaw 202. In contrast, in **FIGS. 9** the over-mold 600 is shown partially disposed over the support member 220, and the core member 250 of the jaw 202, where the over-mold 600 is depicted as transparent to better appreciate how the components of the jaw 202 interface. **FIG. 10** depicts a partial sectional view of the support member 220 and elongated body 230 of the core member 250 of the jaw 202, having the over-mold 600 partially disposed between the support member 220 and the elongated body 230. The over-mold 600 is composed of any suitable non-conductive material, such as a polymer, ceramic, combination of polymeric materials or their like. The over-mold 600 is partially disposed over the support member 220 of the jaw 202, where the support member 220 comprises a proximal end 222 that is not covered by (i.e., free of) the over-mold 600. Further, the over-mold 600 is partially disposed over the core member 250 (e.g., elongated body 230 and the distal portion 240) of the jaw 202, where the conductive seal surface 114 is not covered by (i.e., free of) the over-mold 600. The over-mold 600 may be extruded, molded, coated or any suitable technique on the jaw 202.

In some embodiments, the pair of opposing jaws 202, 204 are configured to selectively conduct an electrosurgical energy for sealing tissue at power levels in a manner similar to that described or illustrated in the '561 patent. The pair of opposing jaws 202, 204 may also comprise material or other design selections as disclosed in the '561 patent and/or the '694 patent. In the illustrated embodiment, the over-mold 600 is illustrated transparently (e.g., **FIGS. 9-12**), although the over-mold 600 may be opaque.

As shown in **FIGS. 6-10****,** the support member 220 of the jaw 202 is spatially separated from the core member 250 (i.e., elongated body 230) of the jaw 202 by a gap 500 (e.g., space, path, opening, break or their like). The gap 500 is configured to mechanically separate/detach and electrically isolate the support member 220 from the core member 250 of the jaw 202, such that the electrical path from the energy source 350 through the wire 320 is directed to the core member 250 (i.e., elongated body 230 and distal portion 240), particularly to the conductive seal surface 114 while electrically isolating the support member 220 of the jaw 202. By electrically and/or thermally insulating the support member 220 of the jaw 202, the support member 220 avoids or minimizes exposure to increase temperatures and/or prolong exposure to heat that could result in material fatigue and failure of the device 100 due to melting, cracking or failure of the over-mold and/or bushing of the jaws. The non-conductive over-mold 600 is further configured to fill in or be disposed within the gap 500. It should be appreciated that other non-conductive material (e.g., polymers, elastomer, foam, or their like) may fill in the gap 500, as alternative to or in addition of the non-conductive over-mold 600, such that the support member 220 is substantially electrically isolated from the core member 250. As shown in **FIG. 5** and **FIGS. 9****-10,** the over-mold 600 provides structural stability to the support member 220 and the core member 250 and maintains them in close proximity to each other.

As better appreciated in the side-view of **FIG. 8****,** the gap 500 is non-linear and may take a tortuous path (e.g., plurality of curves) resulting in the support member 220 and core member 250 (e.g., elongated body portion 230) having a puzzle-piece-like configuration. The jaw 202 includes a surface 225 on the support member 220 and a surface 235 on the core member 250 body portion 230, where surfaces 225 and 235 are opposing to each other, defining the gap 500 therebetween. The surface 225 on the support member 220 of the jaw 202 comprises a mirror-image (e.g., same shape or contour but reverse) to the surface 235 on the core member 250 body portion 230, such that the surfaces 225 and 235 may match each other in a puzzle like fashion (**FIGS. 6-10**). In some embodiments, each of the surfaces 225 and 235 have a width of approximately 0.5 to 2 millimeters. In other embodiments, the width of the surfaces 225 and 235 may vary along a length of the respective surface, and/or the width of the surface 225 may differ from the width of the surface 235. The puzzle-piece-like configuration of the spatially separated support member 220 from the core member 250 defining the gap 500 of the jaw 202, can be used with the embodiments of **FIGS. 1-2B** (e.g., straight jaws with cam-pin) or with the embodiments of **FIGS. 3A-3B** (e.g., curved jaws with links) or in any combination of the jaws, actuation mechanism or components.

The jaw 202 (**FIGS. 6-10**) may be manufactured by laser cutting, molding, metal injection molding, machining, stamping, coining, 3D printing, Electrical Discharge Machining (EDM) or any other suitable techniques, such that the jaw 202 is formed into a separate support member 220 and a separate core member 250; both held together by over-mold 600. The over-mold 600 is partially disposed over the support member 220 and the core member 250 holding them together. In some embodiments, glue may be used between the support member 220 and the core member 250 for additional stability. In these embodiments, the core member 250 (e.g., body portion 230) comprises a proximal end 233 formed by a right angle α, followed by approximately a 20° angle β, followed by approximately a -60° angle φ, followed by an arcuate turn and another approximately 20° angle Ω. The angles α, β, φ and Ω are depicted with respect to the X-Y coordinate system shown in **FIG. 10****.** The transitions between the angles α, β, φ and Ω may comprise gradual or arcuate transitions, as shown in **FIG. 10****;** alternatively, the transitions may be sharp (not shown). The surface 235 disposed on the proximal end 233 of the body portion 230 core member 250 comprises a plurality of sections. As shown, a section 235a is disposed between angles α and β, a section 235b is disposed angles β and φ, a section 235c is disposed angles φ and Ω and a section 235d extends from angle Ω (**FIG. 10**). As previously described, the surface 225 on the support member 220 of the jaw 202 comprises a mirror-image (e.g., same shape or contour but reverse) to the surface 235 on the core member 250. Therefore, a section 225a of the surface 225 is disposed opposite and facing the section 235a of the surface 235; a section 225b of the surface 225 is disposed opposite and facing the section 235b of the surface 235; a section 225c of the surface 225 is disposed opposite and facing the section 235c of the surface 235, and a section 225d of the surface 225 is disposed opposite and facing the section 235d of the surface 235. The surface sections 225 of the support member and the surface sections 235 of the core member 250 comprises respective length that define the gap 500. As further shown in **FIG. 10****,** the gap 500 has a distance "D" of approximately 0.15 to 0.30 millimeters, preferably 0.23 millimeters, between the surfaces 225 and 235 of the jaw 202. The distance "D" of the gap 500 should be sufficiently wide to provide suitable electrical isolation yet sufficiently narrow to avoid making the support member 220 or body portion 230 of the core member 250 too thin (for example, in length "X" in **FIG. 10**), which could compromise mechanical durability of the jaw 202.

**FIG. 9** illustrates the jaw 202 depicting a force vector "V" that is exerted during use of the jaw 202 when a user manipulates the actuation mechanism 300, according to the embodiments of the invention. The opposing pair of jaws 202 and 204 (**FIGS. 3A-3B**) of the device 100 are configured move between the closed (i.e., approximate, clamping or grasping position) to the opened configuration, when the user manipulates the actuation mechanism 300. The opposing pair of jaws 202 and 204 move relative to each other and are pivotally coupled to and/or rotatable about the pivot pin 117 (**FIG. 3B**) disposed within the aperture 237 (**FIG. 9**) to move between the opened and clamping positions of the end effector 205 of the device 100. Further, in the embodiments of **FIGS. 1-2B****,** the opposing pair of jaws 102 and 104 move between the clamping and open positions by the axial translation of the cam-pin 119 coupled to the inner member 111 and disposed within aperture 239 of the jaws. The cam-pin 119 transfers forces to the end effector 105, as the inner member 111 longitudinally translates relative to outer tubular member 113, allowing closing or clamping position (**FIG**. **2A**) and opening (**FIG. 2B**) of the jaws 102 and 104. For example, some of the cam-pin 119 forces are illustrated by a force vector "V" on the jaws 102 or 104 are similar than the force vector "V" depicted in **FIG. 9****.** Additionally, the force vector "V" is exerted in the embodiments of **FIGS. 3A-3B** having the links 162 and 164 interface with the respective jaws 202 and 204, as previously described. In the embodiments of **FIGS. 5-10****,** at least the surface section 225 of the support member 220 has a length "L" that partially defines the gap 500, and such length "L" (shown in **FIGS. 9-10**) is substantially orthogonal to the force vector "V" (shown in **FIG. 9** with graph) exerted when the user manipulates the actuation mechanism 300. As shown in **FIG. 9****,** the surface section 225c of the support member 220 and/or the surface section 235c of the core member 250 are substantially orthogonal to the force vector "V", such that, the force exerted in a region "R" of the jaw 202 is substantially minimized or cancelled by the puzzle-piece-like configuration of the jaw 202. The force vector "V" results in movement of the support member 220 relative to the core member 250, but the puzzle-piece-like configuration of the jaw 202 (**FIGS. 5-10**) is configured to eliminate or minimize shear forces (e.g., at region "R"). It should be appreciated that the shear forces exerted by the cam-pin 119 move the jaw 202 about the pivot pin 117, while some of the forces exerted at the region "R" are avoided or reduced as compared to jaws without gap 500. The pair of opposing jaws 202, 204 are configured to uniformly spread or maximize the compressive forces (e.g., load) across the gap 500 while minimizing the shear forces that could result in relative displacement of the support member 220 and/or the core member 250. In some embodiments, the relative movement of the support member 220 and the core member 250 applies a shear load on the electrically insulating material disposed within the gap 500, where the shear load is substantially less than compressive load. The optimal path and width of the gap depends on the specific loading condition of the device, which may be a function of the length of the support member 220 and whether the jaws are controlled by a cam slot or a round hole. It should be appreciated that the angles α, β, φ and Ω (**FIG. 10**) would be calculated, determined or driven by the location/angle of the vector "V" (**FIG. 9**) in the jaws, such that depending on the applied location/angle of the vector "V", the angles α, β, φ and Ω may be modified to optimize the jaw and support the load applied by vector "V".

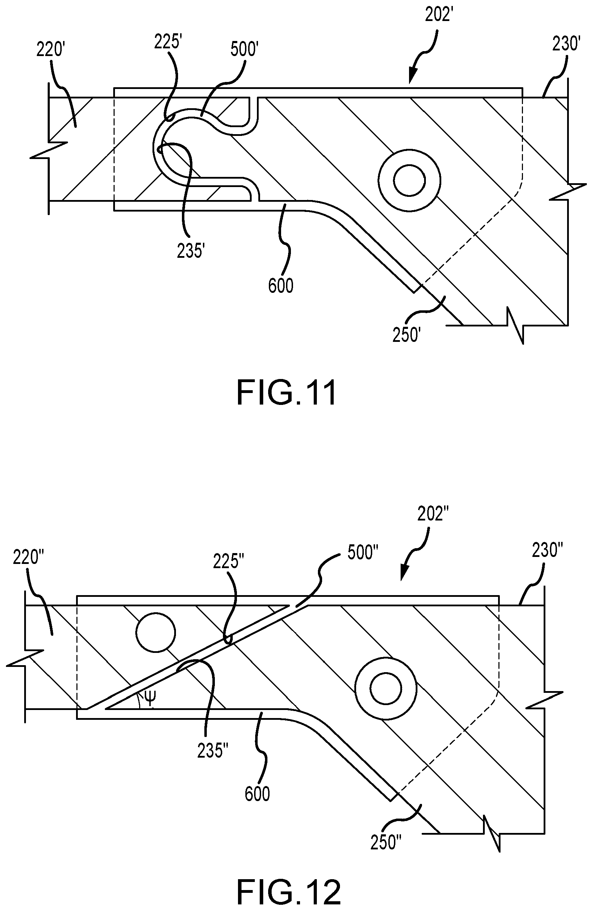

**FIG. 11** and **FIG. 12** illustrate cross-sectional partial views of alternative embodiments of the puzzle-piece-like configuration of the jaw. As shown in **FIG. 11****,** the puzzle-piece-like configuration of a jaw 202' comprises an arcuate or rounded mirror-image surfaces 225' and 235', whereby a support member 220' includes a concave-like contour and a core member 250' body portion 230' comprises a convex-like contour, having an arcuate gap 500' disposed therebetween. As shown in **FIG. 12****,** the puzzle-piece-like configuration of a jaw 202" comprises straight or linear mirror-image surfaces 225" and 235", whereby a support member 220" and a core member 250" body portion 230" comprise a linear contour, having a linear gap 500" disposed therebetween. The linear gap 500" may be disposed in an angle ψ in a range between 10° to 60°.The over-mold 600 is disposed on the jaws 202', 202" and within the gaps 500', 500", similar to gap 500 in the manner previously described. It should be appreciated that other non-conductive material (e.g., elastomer, foam, or their like) may fill in the gaps 500', 500", as alternative to or in addition of the non-conductive over-mold 600.

It should be appreciated that the embodiments of **FIGS 5-10** are the preferred embodiments of the puzzle-piece-like configuration of a jaw, and **FIGS. 11-12** are alternative embodiments. Those skilled in the art can readily recognize that numerous variations and substitutions may be made in the invention, its use and its configuration to achieve substantially the same results as achieved by the embodiments described herein. Accordingly, there is no intention to limit the invention to the disclosed embodiments.

It will be appreciated that the jaws depicted in **FIGS. 5-12** may be used in other suitable medical devices. Further, it will be appreciated that combinations of components, features and functions between the embodiments **FIGS. 1-3** and **FIGS. 5-12** may be made.

Although particular embodiments have been shown and described herein, it will be understood by those skilled in the art that they are not intended to limit the present invention, and it will be obvious to those skilled in the art that various changes, permutations, and modifications may be made (e.g., the dimensions of various parts, combinations of parts) without departing from the scope of the disclosed inventions, which is to be defined only by the following claims. The specification and drawings are, accordingly, to be regarded in an illustrative rather than restrictive sense.

## Claims

1. An electrosurgical device (100), comprising:
a pair of opposing jaws including a first jaw and a second jaw, the first and second jaws (102, 104) configured to move between an open position and a tissue clamping position, each of the first and second jaws (102, 104) comprising an electrically conductive core member (250) and an electrically conductive support member (220),
wherein a distal end of the support member (220) is separated from a proximal end of the core member (250) by a gap (500), with an electrically insulative material (600) disposed in the gap (500) such that the support member (220) is substantially electrically isolated from the core member (250); and
a pin (117), wherein the first and second jaw core members (250) are pivotally coupled to the pin (117) about which the first and second jaws (102, 104) rotate to move between the open position and the tissue clamping position.

2. The electrosurgical device (100) of claim 1, further comprising an actuation mechanism (300) operably coupled to the first and second jaw support members (220) for enabling manipulation of the pair of opposing jaws (102, 104).

3. The electrosurgical device (100) of claim 2, wherein a surface section (225) of at least one of the first and second jaw support members (220) has a length that at least partially defines the gap (500), and wherein the length is substantially orthogonal to a force vector exerted when a user manipulates the actuation mechanism (300).

4. The electrosurgical device (100) of claim 3, wherein the force vector results in movement of the at least one support member (220) relative to the respective core member (250).

5. The electrosurgical device (100) of claim 4, wherein the relative movement of the at least one support member (220) and the respective core member (250) applies a compressive load on the electrically insulating material disposed in the respective gap (500).

6. The electrosurgical device (100) of claim 5, wherein the gap (500) is configured to spread the compressive load substantially uniformly along a length of the gap (500).

7. The electrosurgical device (100) of claim 5, wherein the relative movement of the at least one support member (220) and the respective core member (250) applies a shear load on the electrically insulating material disposed in the gap (500), wherein the shear load is substantially less than compressive load.

8. The electrosurgical device (100) of claim 5, wherein the gap (500) is non-linear.

9. The electrosurgical device (100) of any of claims 3-8, wherein a polymer over-mold is partially disposed over the at least one support member (220) and the respective core member (250).

10. The electrosurgical device (100) of any of claims 1-9, wherein the pin (117) is substantially electrically isolated from the respective first and second jaw core members (250) by a polymer or ceramic bushing.

11. The electrosurgical device (100) of any of claims 2-10, wherein the actuation mechanism (300) comprises a linkage mechanism disposed proximal to the pin (117) and operably coupled to the respective first and second jaw support members (220) for enabling manipulation of the pair of opposing jaws (102, 104).

12. The electrosurgical device (100) of claim 11, wherein the linkage mechanism is electrically isolated from the pin (117) and the first and second jaw core members (250).

## Patentansprüche

1. Elektrochirurgische Vorrichtung (100), umfassend:
ein Paar gegenüberliegender Backen, das eine erste Backe und eine zweite Backe einschließt, wobei die erste und die zweite Backe (102, 104) konfiguriert sind, sich zwischen einer offenen Position und einer Gewebeklemmposition zu bewegen, wobei jede der ersten und der zweiten Backe (102, 104) ein elektrisch leitendes Kernelement (250) und ein elektrisch leitendes Stützelement (220) umfasst,
wobei ein distales Ende des Stützelements (220) von einem proximalen Ende des Kernelements (250) durch einen Spalt (500) getrennt ist, wobei ein elektrisch isolierendes Material (600) in dem Spalt (500) angeordnet ist, sodass das Stützelement (220) im Wesentlichen elektrisch von dem Kernelement (250) isoliert ist; und
einen Stift (117), wobei das erste und das zweite Backenkernelement (250) schwenkbar mit dem Stift (117) gekoppelt sind, um welchen sich die erste und die zweite Backe (102, 104) drehen, um sich zwischen der offenen Position und der Gewebeklemmposition zu bewegen.

2. Elektrochirurgische Vorrichtung (100) nach Anspruch 1, ferner umfassend einen Betätigungsmechanismus (300), der betriebsmäßig an das erste und das zweite Backenstützelement (220) zum Ermöglichen einer Manipulation des Paars gegenüberliegender Backen (102, 104) gekoppelt ist.

3. Elektrochirurgische Vorrichtung (100) nach Anspruch 2, wobei ein Oberflächenabschnitt (225) von zumindest einem von dem ersten und dem zweiten Backenstützelement (220) eine Länge aufweist, die zumindest teilweise den Spalt (500) definiert, und wobei die Länge im Wesentlichen orthogonal zu einem Kraftvektor ist, der ausgeübt wird, wenn ein Benutzer den Betätigungsmechanismus (300) manipuliert.

4. Elektrochirurgische Vorrichtung (100) nach Anspruch 3, wobei der Kraftvektor zu einer Bewegung des zumindest einen Stützelements (220) relativ zu dem jeweiligen Kernelement (250) führt.

5. Elektrochirurgische Vorrichtung (100) nach Anspruch 4, wobei die relative Bewegung des zumindest einen Stützelements (220) und des jeweiligen Kernelements (250) eine Druckbelastung auf das elektrisch isolierende Material ausübt, das in dem jeweiligen Spalt (500) angeordnet ist.

6. Elektrochirurgische Vorrichtung (100) nach Anspruch 5, wobei der Spalt (500) konfiguriert ist, um die Druckbelastung im Wesentlichen gleichmäßig entlang einer Länge des Spalts (500) zu verteilen.

7. Elektrochirurgische Vorrichtung (100) nach Anspruch 5, wobei die relative Bewegung des zumindest einen Stützelements (220) und des jeweiligen Kernelements (250) eine Scherbelastung auf das elektrisch isolierende Material ausübt, das in dem Spalt (500) angeordnet ist, wobei die Scherbelastung wesentlich geringer als eine Druckbelastung ist.

8. Elektrochirurgische Vorrichtung (100) nach Anspruch 5, wobei der Spalt (500) nichtlinear ist.

9. Elektrochirurgische Vorrichtung (100) nach einem der Ansprüche 3-8, wobei eine Polymerumspritzung teilweise über dem zumindest einen Stützelement (220) und dem jeweiligen Kernelement (250) angeordnet ist.

10. Elektrochirurgische Vorrichtung (100) nach einem der Ansprüche 1-9, wobei der Stift (117) im Wesentlichen elektrisch von dem jeweiligen ersten und zweiten Backenkernelement (250) durch eine Polymer- oder Keramikbuchse isoliert ist.

11. Elektrochirurgische Vorrichtung (100) nach einem der Ansprüche 2-10, wobei der Betätigungsmechanismus (300) einen Verbindungsmechanismus umfasst, der proximal zu dem Stift (117) angeordnet und betriebsmäßig an das jeweilige erste und zweite Backenstützelement (220) zum Ermöglichen einer Manipulation des Paars gegenüberliegender Backen (102, 104) angeordnet ist.

12. Elektrochirurgische Vorrichtung (100) nach Anspruch 11, wobei der Verbindungsmechanismus elektrisch von dem Stift (117) und dem ersten und zweiten Backenkernelement (250) isoliert ist.

## Revendications

1. Dispositif électrochirurgical (100) comprenant :
une paire de mâchoires opposées comprenant une première mâchoire et une seconde mâchoire, les première et seconde mâchoires (102, 104) étant conçues pour se déplacer entre une position ouverte et une position de serrage de tissu, chacune des première et seconde mâchoires (102, 104) comprenant un élément de noyau électroconducteur (250) et un élément de support électroconducteur (220),
une extrémité distale de l'élément de support (220) étant séparée d'une extrémité proximale de l'élément de noyau (250) par un espace (500), un matériau électriquement isolant (600) étant disposé dans l'espace (500) de sorte que l'élément de support (220) soit sensiblement électriquement isolé de l'élément de noyau (250) ; et
une broche (117), lesdits premier et second éléments de noyau de mâchoire (250) étant couplés pivotants à la broche (117) autour de laquelle les première et seconde mâchoires (102, 104) tournent pour se déplacer entre la position ouverte et la position de serrage de tissu.

2. Dispositif électrochirurgical (100) selon la revendication 1, comprenant en outre un mécanisme d'actionnement (300) couplé fonctionnellement aux premier et second éléments de support de mâchoire (220) pour permettre la manipulation de la paire de mâchoires opposées (102, 104).

3. Dispositif électrochirurgical (100) selon la revendication 2, une section de surface (225) d'au moins l'un des premier et second éléments de support de mâchoire (220) présentant une longueur qui définit au moins partiellement l'espace (500), et ladite longueur étant sensiblement orthogonale à un vecteur de force exercé lorsqu'un utilisateur manipule le mécanisme d'actionnement (300).

4. Dispositif électrochirurgical (100) selon la revendication 3, ledit vecteur de force entraînant le déplacement dudit au moins un élément de support (220) par rapport à l'élément de noyau respectif (250).

5. Dispositif électrochirurgical (100) selon la revendication 4, ledit déplacement relatif dudit au moins un élément de support (220) et de l'élément de noyau respectif (250) appliquant une charge de compression sur le matériau électriquement isolant disposé dans l'espace respectif (500).

6. Dispositif électrochirurgical (100) selon la revendication 5, ledit espace (500) étant conçu pour répartir la charge de compression de manière sensiblement uniforme le long d'une longueur de l'espace (500).

7. Dispositif électrochirurgical (100) selon la revendication 5, ledit déplacement relatif dudit au moins un élément de support (220) et de l'élément de noyau respectif (250) appliquant une charge de cisaillement sur le matériau électriquement isolant disposé dans l'espace (500), ladite charge de cisaillement étant sensiblement inférieure à la charge de compression.

8. Dispositif électrochirurgical (100) selon la revendication 5, ledit espace (500) étant non linéaire.

9. Dispositif électrochirurgical (100) selon l'une quelconque des revendications 3 à 8, un surmoulage de polymère étant partiellement disposé sur ledit au moins un élément de support (220) et l'élément de noyau respectif (250).

10. Dispositif électrochirurgical (100) selon l'une quelconque des revendications 1 à 9, ladite broche (117) étant sensiblement électriquement isolée des premier et second éléments de noyau de mâchoire respectifs (250) par une douille en polymère ou en céramique.

11. Dispositif électrochirurgical (100) selon l'une quelconque des revendications 2 à 10, ledit mécanisme d'actionnement (300) comprenant un mécanisme de liaison disposé à proximité de la broche (117) et couplé fonctionnellement aux premier et second éléments de support de mâchoire respectifs (220) pour permettre la manipulation de la paire de mâchoires opposées (102, 104).

12. Dispositif électrochirurgical (100) selon la revendication 11, ledit mécanisme de liaison étant électriquement isolé de la broche (117) et des premier et second éléments de noyau de mâchoire (250).
